# EUROPEAN PATENT SPECIFICATION

(11) **EP 2 077 787 B1**
(45) Date of publication and mention of the grant of the patent: **01.07.2015**
(21) Application number: 07863571.1
(22) Date of filing: 26.10.2007
(51) Int. Cl.: A61B 18/14

(54) **BIPOLAR ABLATION PROBE HAVING POROUS ELECTRODES FOR DELIVERING ELECTRICALLY CONDUCTIVE FLUID**
BIPOLARE ABLATIONSSONDE MIT PORÖSEN ELEKTRODEN ZUR AUSGABE EINER ELEKTRISCH LEITENDEN FLÜSSIGKEIT
SONDE D'ABLATION BIPOLAIRE COMPORTANT DES ÉLECTRODES POREUSES PERMETTANT LA DÉLIVRANCE D'UN FLUIDE ÉLECTROCONDUCTEUR

(30) Priority: 01.11.2006 US 863940 P
(43) Date of publication of application: 15.07.2009
(73) Proprietor: Bovie Medical Corporation, St Petersburg FL 33710 (US)
(72) Inventor: RIOUX, Robert F., Ashland, Massachusetts 01721 (US); LEVENDUSKY, Joe, Groton, Massachusetts 01450 (US); BEAN, Jeffrey, Fitchburg, Massachusetts 01420 (US)
(74) Representative: McDonough, Jonathan
(86) International application number: PCT/US2007/082721
(87) International publication number: WO 2008/057805

(56) References cited:
- EP-A- 0 895 755
- EP-A- 0 895 756
- US-A1- 2002 026 187
- US-A1- 2003 009 164

## Description

### Field Of Invention

The invention relates to tissue ablation devices, and particularly to ablation devices used for achieving tissue resection.

### Background

Monopolar electrosurgical devices typically comprise an electrosurgical probe having a first or "active" electrode that is electrically coupled to an electrosurgical generator. A second or "return" electrode, having a much larger surface area than the active electrode, is positioned in contact with the skin of the patient. For the bipolar modality, no return electrode is used. Instead, a second electrode is closely positioned adjacent to the first electrode, with both electrodes being attached to an electrosurgical probe. When the generator is activated, electrical current arcs from the end of the first electrode to the end of the second electrode, flowing through the intervening tissue.

Whether arranged in a monopolar or bipolar fashion, the active electrode may be operated to either cut tissue or coagulate tissue. When used to cut tissue, the electrical arcing and corresponding current flow results in a highly intense, but localized heating, sufficient enough to break intercellular bonds, resulting in tissue severance. When used to coagulate tissue, the electrical arcing results in a low level current that denatures cells to a sufficient depth without breaking intercellular bonds, i.e., without cutting the tissue.

Whether tissue is cut or coagulated mainly depends on the geometry of the active electrode and the nature of the electrical energy delivered to the electrode. In general, the smaller the surface area of the electrode in proximity to the tissue, the greater the current density (i.e., the amount of current distributed over an area) of the electrical arc generated by the electrode, and thus the more intense the thermal effect, thereby cutting the tissue. In contrast, the greater the surface area of the electrode in proximity to the tissue, the less the current density of the electrical arc generated by the electrode, thereby coagulating the tissue. Thus, if an electrode having both a broad side and a narrow side is used, e.g., a spatula, the narrow side of the electrode can be placed in proximity to the tissue in order to cut it, whereas the broad side of the electrode can be placed in proximity to the tissue in order to coagulate it. With respect to the characteristics of the electrical energy, as the crest factor (peak voltage divided by root mean squared (RMS)) of the electrical energy increases, the resulting electrical arc generated by the electrode tends to have a tissue coagulation effect. In contrast, as the crest factor of the electrical energy decreases, the resulting electrical arc generated by the electrode tends to have a cutting effect. The crest factor of the electrical energy is typically controlled by controlling the duty cycle of the electrical energy. For example, to accentuate tissue cutting, the electrical energy may be continuously applied to increase its RMS average to decrease the crest factor. In contrast, to accentuate tissue coagulation, the electrical energy may be pulsed (e.g., at a 10 percent duty cycle) to decrease its RMS average to increase the crest factor.

Notably, some electrosurgical generators are capable of being selectively operated in so-called "cutting modes" and "coagulation modes." This, however, does not mean that the active electrode that is connected to such electrosurgical generators will necessarily have a tissue cutting effect if operated in the cutting mode or similarly will have a tissue coagulation effect if operated in the coagulation mode, since the geometry of the electrode is the most significant factor in dictating whether the tissue is cut or coagulated. Thus, if the narrow part of an electrode is placed in proximity to tissue and electrical energy is delivered to the electrode while in a coagulation mode, the tissue may still be cut.

There are many medical procedures in which tissue is cut or carved away for diagnostic or therapeutic reasons. For example, during hepatic transection, one or more lobes of a liver containing abnormal tissue, such as malignant tissue or fibrous tissue caused by cirrhosis, are cut away. There exists various modalities, including mechanical, ultrasonic, and electrical (which includes RF energy), that can be used to effect resection of tissue. Whichever modality is used, extensive bleeding can occur, which can obstruct the surgeon's view and lead to dangerous blood loss levels, requiring transfusion of blood, which increases the complexity, time, and expense of the resection procedure. To prevent extensive bleeding, hemostatic mechanisms, such as blood inflow occlusion, coagulants (e.g., Surgicel™ or Tisseel™), and energy coagulation (e.g., electrosurgical coagulation or argon-beam coagulation), can be used.

In the case where an electrosurgical coagulation means is used, the bleeding can be treated or avoided by coagulating the tissue in the treatment areas with an electro-coagulator that applies a low level current to denature cells to a sufficient depth without breaking intercellular bonds, i.e., without cutting the tissue. Because of their natural coagulation capability, ease of use, and ubiquity, electrosurgical modalities are often used to resect tissue.

During a typical electrosurgical resection procedure, electrical energy can be conveyed from an electrode along a resection line in the tissue. The electrode may be operated in a manner that incises the tissue along the resection line, or coagulates the tissue along the resection line, which can then be subsequently dissected using the same coagulation electrode or a separate tissue dissector to gradually separate the tissue. In the case where an organ is resected, application of RF energy divides the parenchyma, thereby skeletalizing the organ, i.e., leaving vascular tissue that is typically more difficult to cut or dissect relative to the parenchyma.

When a blood vessel is encountered, RF energy can be applied to shrink the collagen in the blood vessel, thereby closing the blood lumen and achieving hemostasis. The blood vessel can then be mechanically transected using a scalpel or scissors without fear of blood loss. In general, for smaller blood vessels less than 3 mm in diameter, hemostasis may be achieved within 10 seconds, whereas for larger blood vessels up to 5 mm in diameter, the time required for hemostasis increases to 15-20 seconds. During or after resection of the tissue, RF energy can be applied to any "bleeders" (i.e., vessels from which blood flows or oozes) to provide complete hemostasis for the resected organ.

Although the application of electrically conductive fluid to the electrosurgery site generally increases the efficiency of the RF energy application, energy applied to an electrode may rapidly diffuse into fluid that has accumulated and into tissue that has already been removed. As a result, if the fluid and removed tissue is not effectively aspirated from the tissue site, the electrosurgery may either be inadequately carried out, or a greater than necessary amount of energy must be applied to the electrode to perform the surgery. Increasing the energy used during electrosurgery increases the chance that adjacent healthy tissues may be damaged. At the same time that fluid accumulation is avoided, care must be taken to ensure that fluid is continuously flowed to the tissue site to ensure that tissue charring does not take place. For example, if flow of the fluid is momentarily stopped, e.g., if the port on the fluid delivery device becomes clogged or otherwise occluded, RF energy may continue to be conveyed from the electrode, thereby resulting in a condition where tissue charring may occur.

EP 0895756 discloses a tissue ablation probe according to the preamble of claim 1.

### Summary of the Invention

In accordance with the invention, a tissue ablation probe is provided. The ablation probe comprises an elongated probe shaft (e.g., a rigid shaft), at least one fluid perfusion lumen longitudinally extending through the probe shaft, and a plurality of electrodes carried by the distal end of the probe shaft. In one embodiment, the probe shaft comprises a plurality of tubes on which the electrodes are respectively mounted, and the perfusion lumen(s) comprise a plurality of lumens longitudinally extending through the tubes. In another embodiment, the electrodes may be configured in a multi-polar arrangement. The electrodes have a substantially co-extensive surface. According to the invention, the substantially co-extensive surface is a distal-facing surface, which is tapered. The co-extensive surface may be a planar surface or a curved surface.

Each of the electrodes includes a porous structure in fluid communication with the perfusion lumen(s). The porous structure of each of the electrodes is composed of an electrically conductive material. In one embodiment, the porous structure has pores with effective diameters in the range of 1-50 microns. In another embodiment, the porous structure has interconnecting pores. Optionally, the ablation probe may comprise a connector assembly mounted to the proximal end of the probe shaft, wherein the connector assembly comprises at least one port in fluid communication with the perfusion lumen(s).

In accordance with another arrangement, a tissue ablation probe is provided. The ablation probe comprises an elongated probe shaft (e.g., a rigid shaft), at least one fluid perfusion lumen longitudinally extending through the probe shaft, and first and second electrodes carried by the distal end of the probe shaft. In one example, the probe shaft comprises a plurality of tubes on which the electrodes are respectively mounted, and the perfusion lumen(s) comprise a plurality of lumens longitudinally extending through the tubes. In another example, the electrodes may be configured in a bipolar arrangement. The electrodes have distal-facing surfaces that extend within the same plane. In one example, the plane is angled relative to the longitudinal axis of the probe shaft. Each of the electrodes includes a porous structure in fluid communication with the perfusion lumen(s) and optionally a connector assembly mounted to the proximal end of the probe shaft, wherein the connector assembly comprises at least one port in fluid communication with the perfusion lumen(s). The detailed features of the porous structure may be the same as those previously described.

### Brief Description Of The Drawings

The drawings illustrate the design and utility of embodiments of the invention, in which similar elements are referred to by common reference numerals, and in which:
**Fig. 1** is a plan view of a tissue coagulation/resection system constructed in accordance with one embodiment of the invention;
**Fig. 2** is a partially cutaway, perspective, view of the distal end of a tissue ablation probe used in the system of **Fig. 1**;
**Fig. 3** is a perspective view of a distal collar used to integrate the tissue ablation probe of **Fig. 2**;
**Fig. 4** is a partially cutaway, perspective, view of the distal end of an alternative embodiment of a tissue ablation probe that can be used in the system of **Fig. 1****;**
**Fig. 5** is a partially cutaway, perspective, view of the distal end of another alternative embodiment of a tissue ablation probe that can be used in the system of **Fig. 1****;**
**Fig. 6** is a partially cutaway, perspective, view of the distal end of yet another alternative embodiment of a tissue ablation probe that can be used in the system of **Fig. 1**;
**Fig. 7** is a close-up side view of an electrode used in the tissue ablation probe of **Fig. 2**;
**Fig. 8A** is a perspective view of tissue having an unhealthy tissue portion to be resected from a healthy tissue portion, wherein tissue along a resection line has been coagulated using the tissue coagulation/resection system of **Fig. 1****;**
**Fig. 8B** is a perspective view of the tissue of **Fig. 8A****,** wherein tissue along the resection line has been separated using the tissue coagulation/resection system of **Fig. 1**;
**Fig. 8C** is a perspective view of the tissue of **Fig. 8A**, wherein anatomical vessels have been exposed along the resection line by the tissue coagulation/resection system of **Fig. 1**; and
**Fig. 8D** is a perspective of the tissue of **Fig. 8A**, wherein the unhealthy tissue portion has been completely resected from the healthy portion using the tissue coagulation/resection system of **Fig. 1**.

### Detailed Description Of The Illustrated Embodiments

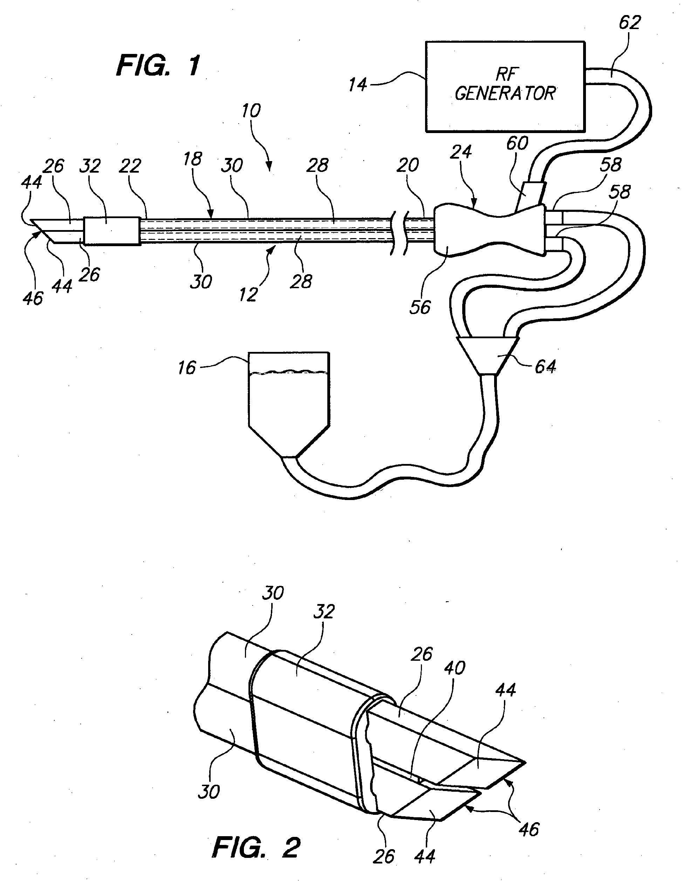

**Fig. 1** illustrates a tissue resection system 10 constructed in accordance with a embodiment of the invention. The tissue resection system 10 generally comprises a tissue ablation probe 12 configured for coagulating and resecting tissue, an ablation energy source, and in particular a radio frequency (RF) generator 14, configured for supplying RF energy to the tissue resection probe 12 in a controlled manner, and an electrically conductive fluid source, and in particular a saline bag 16, configured for supplying electrically conductive fluid (e.g., saline) to the probe 12 to provide an electrically conductive path for the RF energy from the probe 12 to the tissue to be coagulated/resected.

The ablation probe 12 generally comprises an elongated probe shaft 18 having a proximal end 20, a distal end 22, a handle assembly 24 mounted to the proximal shaft end 20, a pair of electrodes 26 mounted to the distal shaft end 22, and a pair of fluid perfusion lumens 28 (shown in phantom) extending through the probe shaft 18 between the proximal shaft end 20 and the probe distal end 22. In the illustrated embodiment, the probe shaft 18 is rigid, thereby providing maximum control at the distal end 22 of the probe shaft 18. The probe shaft 18 is composed of a suitable material, such as plastic, metal or the like, and has a suitable length, typically in the range from 5 cm to 30 cm, preferably from 10 cm to 20 cm. If composed of an electrically conductive material, the probe shaft 18 is preferably covered with an insulative material (not shown). The probe shaft 18 has an outside diameter consistent with its intended use.

Referring further to **Fig. 2**, the probe shaft 18 includes a pair of elongated probe tubes 30 to which the electrodes 26 are respectively coupled in a side-by-side arrangement. In this case, separate fluid perfusion lumens 28 respectively extend through the lengths of the probe tubes 30. The distal ends of the probe tubes 30 are mounted within apertures (not shown) formed in the proximal ends of the electrodes 26. The distal ends of the probe tubes 30 may have reduced diameters to facilitate mounting within the apertures of the electrodes 26. A bonding material may be used to affix the electrodes 26 to the probe tubes 30.

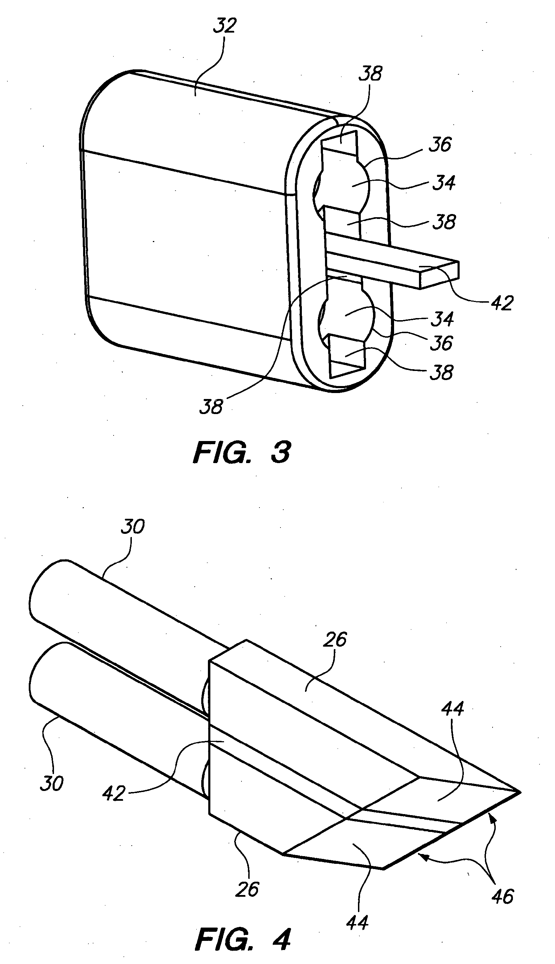

Referring further to **Fig. 3**, the ablation probe 12 comprises a distal collar 32 that integrates the probe tubes 30 and electrodes 26 together. In particular, the distal collar 32 includes a pair of lumens 34, the proximal ends in which the probe tubes 30 are respectively retained, and the distal ends in which the electrodes 26 are respectively retained. The cross-section of the outer periphery of the distal collar 32 is oval or elongated to more easily house the lumens 34. The cross-section of each of the collar lumens 34 conforms to the cross-sections of the probe tubes 30 and electrodes 26. In the illustrated embodiment, each tube 30 has a circular cross-section and each electrode 26 has a rectangular cross-section. In this case, each collar lumen 34 has a generally circular cross-section 36 with opposing rectangular extensions 38. Thus, the circular cross-section 36 at the proximal end of each collar lumen 34 receives the distal end of the respective tube 30, while the rectangular cross-section 36 at the distal end of each collar lumen 34 receives the proximal end of the respective electrode 26.

The probe tubes 30 and electrodes 26 may be affixed within the collar lumens 34 using suitable means, such as bonding. Thus, the probe tubes 30 and electrodes 32 are retained within the distal collar 32, so that they are inhibited from separating or rotating relative to each other. The distal collar 32 may be composed of any suitably electrically insulative material, such as polycarbonate. As will be described in further detail below, the probe tubes 30 are proximally retained within the handle assembly 24. The electrodes 26 are electrically and mechanically isolated from each other by an electrically insulative spacer 40 distally extending from the distal collar 32. The spacer 40 may be composed of the same material as the distal collar 32 and may be molded with the distal collar 32 as a unibody structure. Alternatively, instead of using the distal collar 32, the electrodes 26 can be bonded together via an electrically insulative material 42, as illustrated in **Fig. 4****.** Thus, the electrically insulative material 42 serves as both an electrically insulative spacer and a means for integrating the probe tubes 30 and electrodes 26 together.

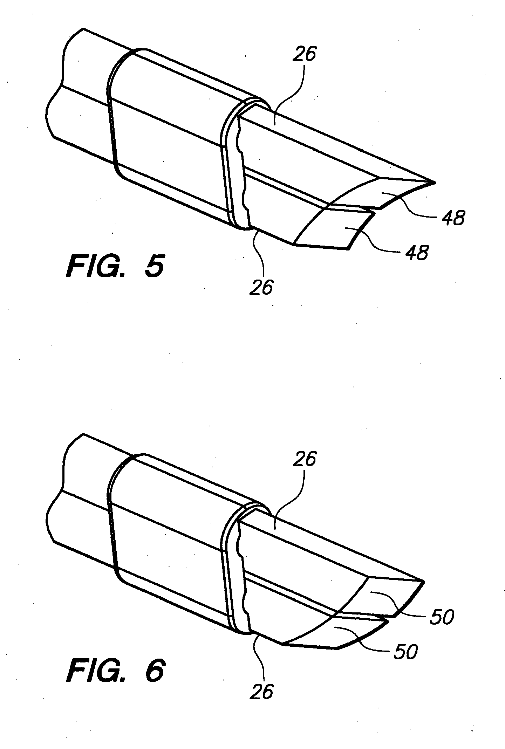

Significantly, whichever manner the probe tubes 30 and electrodes 26 are integrated, the electrodes 26 have distal-facing surfaces 44 that form a substantially co-extensive surface 46 that conforms to the tissue surface. For the purposes of this specification, two different surfaces form a substantially co-extensive surface if the respective surfaces effectively form a continuous surface (with the spacing therebetween being negligible) when placed in contact with a tissue surface. In the embodiments illustrated in **Figs. 2** and **4**, the distal-facing surfaces 44 are flat and are disposed in the same plane. Alternatively, the electrodes 26 have distal-facing concave surfaces 48 (**Fig. 5**) or distal-facing convex surfaces 50 (**Fig**. **6**) that form a substantially co-extensive surface. In the illustrated embodiments, the co-extensive surfaces of the electrodes 26 are tapered, thereby increasing the area of the co-extensive surface. In addition, the tapered co-extensive surface facilitates its flush placement with tissue given a natural angled orientation of the ablation probe 12. Notably, the distal-facing surfaces 44, 48, 50 of the electrodes 26 have a size that is consistent with effecting tissue coagulation. Alternatively, the distal-facing surfaces 44, 48, 50 have a smaller size that is consistent with effecting tissue cutting.

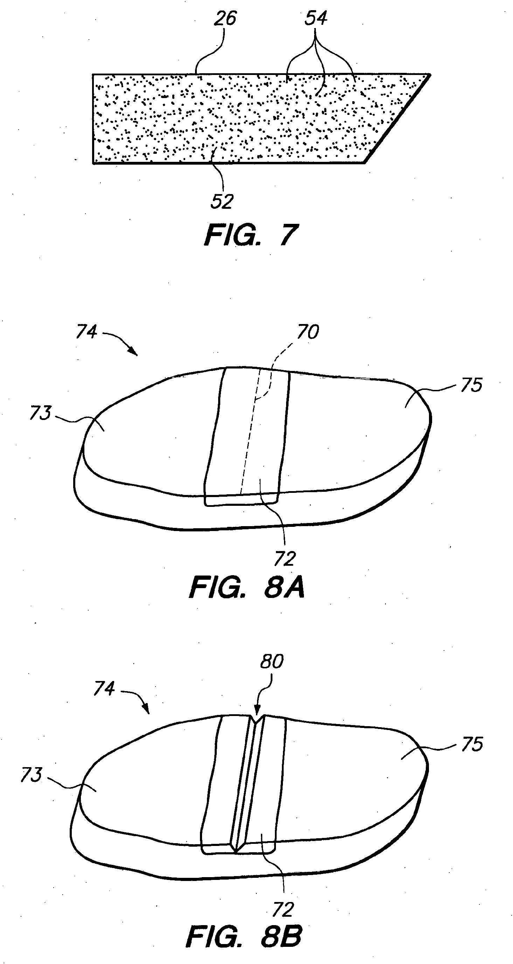

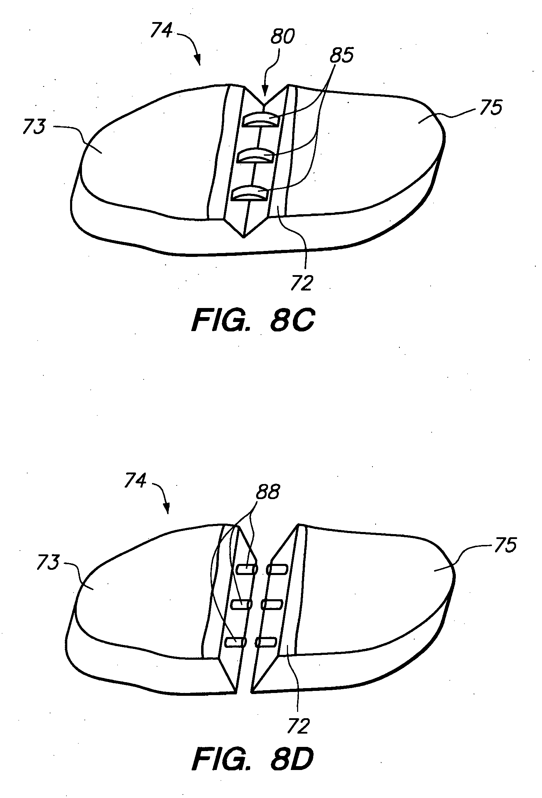

Referring to **Figs. 7** and **8**, each of the electrodes 26 is composed of porous structure 52 that renders the electrode 26 pervious to the passage of fluid, thereby facilitating the uniform distribution of an electrically conductive fluid into the tissue during the ablation process. The porous structure 52 allows fluid to not only pass around the electrode 26 on the outer surface of the electrode 26, but also allows fluid to pass through the electrode 26. In addition to providing a more uniform distribution of fluid, the porous structure 52, tissue is less apt to stick to the surfaces of the electrode 26.

To this end, the porous structure 52 comprises a plurality of pores 54 that are in fluid communication with the perfusion lumens 28 extending through the probe tubes 30. In the illustrated embodiment, the pores 54 are interconnected in a random, tortuous, interstitial arrangement in order to maximize the porosity of the electrodes 26. The porous structure 52 may be microporous, in which case, the effective diameters of the pores 54 will be in the 0.05-20 micron range, or the porous structure 52 may be macroporous, in which case, the effective diameters of the pores 54 will be in the 20-2000 micron range. A preferred pore size will be in the 1-50 micron range. The porosity of the porous structure 52, as defined by the pore volume over the total volume of the structure, is preferably in the 20-80 percent range, and more preferably within the 30-70 percent range. Naturally, the higher the porosity, the more freely the fluid will flow through the electrodes 26. Thus, the designed porosity of the porous structure 52 will ultimately depend on the desired flow of the fluid. Of course, the porous structure 52 should not be so porous as to unduly sacrifice the structural integrity of the electrodes 26.

Thus, it can be appreciated that the pervasiveness of the pores 54 allows the fluid to freely flow from the perfusion lumens 28, through the thickness of the electrodes 26, and out to the adjacent tissue. Significantly, this free flow of fluid will occur even if several of the pores 54 have been clogged with material, such as tissue. For purposes of ease in manufacturability, the entirety of the electrodes 26 is composed of the porous structure 52. Alternatively, only the portion of the electrodes 26 that will be adjacent the ablation region (e.g., the distal portion of the electrodes 26) is composed of the porous structure 52. Preferably, the porous structure 52 provides for the wicking (i.e., absorption of fluid by capillary action) of fluid into the pores 54 of the porous structure 52. To promote the wicking of fluid into the porous structure, the porous structure may be hydrophilic.

The porous structure 52 is preferably composed of a metallic material, such as stainless steel, titanium, or nickel-chrome. While each electrode 26 is preferably composed of an electrically conductive material, the electrode 26 may alternatively be composed of a non-metallic material, such as porous polymer or ceramic. While the porous polymers and ceramics are generally non-conductive, they may be used to conduct electrical energy to the tissue by virtue of the conductive fluid within the interconnected pores.

In one embodiment, the porous structure 52 is formed using a sintering process, which involves compacting a plurality of particles (preferably, a blend of finely pulverized metal powers mixed with lubricants and/or alloying elements) into the shape of the electrode 26, and then subjecting the blend to high temperatures. When compacting the particles, a controlled amount of the mixed powder is automatically gravity-fed into a precision die and is compacted, usually at room temperature at pressures as low as 10 or as high as 60 or more tons/inch² (138 to 827 MPa), depending on the desired porosity of the electrode 26. The compacted powder will have the shape of the electrode 26 once it is ejected from the die, and will be sufficiently rigid to permit in-process handling and transport to a sintering furnace. Other specialized compacting and alternative forming methods can be used, such as powder forging, isostatic pressing, extrusion, injection molding, and spray forming.

During sintering, the unfinished electrode 26 is placed within a controlled-atmosphere furnace, and is heated to below the melting point of the base metal, held at the sintering temperature, and then cooled. The sintering transforms the compacted mechanical bonds between the power particles to metallurgical bonds. The interstitial spaces between the points of contact will be preserved as pores. The amount and characteristics of the porosity of the structure 52 can be controlled through powder characteristics, powder composition, and the compaction and sintering process.

Porous structures can be made by methods other than sintering. For example, pores may be introduced by mechanical perforation, by the introduction of pore producing agents during a matrix forming process, or through various phase separate techniques. Also, the porous structure may be composed of a ceramic porous material with a conductive coating deposited onto the surface, e.g., by using ion beam deposition or sputtering.

Referring back to **Fig. 1****,** the handle assembly 24 comprises a handle 56 that is preferably composed of a durable and rigid material, such as medical grade plastic, and is ergonomically molded to allow a physician to more easily manipulate the ablation probe 12. The proximal ends of the probe tubes 30 are mounted within apertures (not shown) formed at the distal end of the handle 56. The handle assembly 24 further comprises a pair of perfusion inlet ports 58 (e.g., male luer connectors) in fluid communication with the perfusion lumens 28 extending through the probe tubes 30. The handle assembly 24 further comprises a radio frequency (RF) electrical port 60 in electrical communication with the electrodes 26 in a bipolar arrangement. That is, current will pass between the electrodes 26, thereby having injurious effect on the tissue in adjacent the electrodes 26. In one embodiment, the electrical port 60 is in electrical communication with the electrodes 26 via the walls of the probe tubes 30 (if composed of an electrically conductive material). In other embodiments, the electrical port 60 may be in electrical communication with the electrodes 26 via RF wires (not shown) extending through the probe tubes 30.

Referring back to **Fig. 1****,** the RF generator 14 is electrically connected to the electrical port 60 of the handle assembly 24 via an RF cable 62, which as previously described, is indirectly electrically coupled to the electrodes 26 via the probe tubes 30 or wires (not shown). The RF generator 14 may be a conventional RF power supply that operates at a frequency in the range from 200 KHz to 9.5 MHz, with a conventional sinusoidal or non-sinusoidal wave form. Such power supplies are available from many commercial suppliers, such as Valleylab, Aspen, and Bovie. Most general purpose electrosurgical power supplies, however, operate at higher voltages and powers than would normally be necessary or suitable for tissue ablation. Thus, such power supplies would usually be operated at the lower ends of their voltage and power capabilities. More suitable power supplies will be capable of supplying an ablation current at a relatively low voltage, typically below 150V (peak-to-peak), usually being from 50V to 100V. The power will usually be from 20W to 200W, usually having a sine wave form, although other wave forms would also be acceptable. Power supplies capable of operating within these ranges are available from commercial vendors, such as Boston Scientific Corporation of San Jose, California, who markets these power supplies under the trademarks RF2000™ (100W) and RF3000™ (200W).

In the illustrated embodiment, the fluid source 16 takes the form of a saline bag connected to the fluid infusion ports 58 via a Y-conduit 64. The saline bag 16 is conventional and is of a suitable size, e.g., 200 ml. In the illustrated embodiment, the saline is 0.9% saline. Thus, it can be appreciated the saline bag 16 can be raised above the patient a sufficient height to provide the head pressure necessary to convey the saline under pressure through the Y-conduit 64, into the fluid infusion ports 58, through the perfusion lumens 28, and out of the electrodes 26. Alternatively, rather than a saline bag, the fluid source may take the form of a pump assembly or a syringe.

Having described the general structure and function of the tissue resection system 10, its operation in resecting tissue will be described. The tissue may be located anywhere in the body where resection may be beneficial. Most commonly, the tissue will contain a solid tumor within an organ of the body, such as the liver, kidney, pancreas, breast, prostrate (not accessed via the urethra), and the like. In this case, an unhealthy tissue portion, e.g., a cancerous portion containing a tumor, e.g., a lobe of a liver, may be resected from the healthy portion of the tissue. For purposes of better understanding the invention, a method to access to the tissue may be accomplished through a surgical opening to facilitate movement of the resection probe within the patient as well as to facilitate removal of the resected tissue from the patient. However, access to the tissue may alternatively be provided through a percutaneous opening, e.g., laparoscopically, in which case, the tissue resection probe can be introduced into the patient through a cannula, and the removed tissue may be minsilated and aspirated from the patient through the cannula.

The operation of the tissue resection system 10 is described in resecting unhealthy portion of tissue to be removed from a patient, which has a tumor, from a healthy portion of tissue to be retained within the patient. First, the RF generator 14 and associated cable 62 are connected to the electrical connector 60 on the probe 12, and the saline bag 16 and associated Y-conduit 64 are connected to the perfusion ports 58 on the probe 12. As a result, the saline is conveyed under positive pressure, through the Y-conduit 64, and into the perfusion ports 58. The saline then travels through the fluid perfusion lumens 28 within the probe tubes 30, and into contact with the electrodes 26. The saline is conveyed out of the pores 54 of the electrodes 26 and into contact with the outer surface of the electrodes 26.

Next, and for purposes of better understanding the invention, the resection probe 12 is manipulated, such that the coagulation electrode is moved in proximity to the tissue along opposite lateral sides of a resection line, and RF energy is conveyed from the RF generator 14 to the electrodes 26 (in particular, between the "positive" electrode 26 and "negative" electrode), resulting in the coagulation of the tissue adjacent a resection line, as illustrated in **Fig. 8A****.** In particular, electrical energy is conveyed between the electrodes 26 through the tissue 74 along the resection line 70, thereby coagulating a band of tissue 72 that straddles the resection line to thereby resect the unhealthy tissue portion 75 from the healthy tissue portion 73. The electrodes 26 may be placed in direct contact with the tissue 74, or alternatively, if the voltage is great enough, may be moved just above the tissue 74, such that arcing occurs between the electrodes 26 and tissue 74.

Next, the coagulated tissue 72 along the resection line is separated, as illustrated in **Fig. 8B**. In the illustrated method, the coagulated tissue 72 is separated 80 by continuing to move the electrodes 26 along the resection line 70. The RF energy conveyed between the tissue 74 and electrodes 26 provides most, if not all, of the tissue resection energy-although mechanical pressure may be applied between the electrodes 26 (or other mechanical resection member) and the tissue 74 to facilitate tissue resection. The tissue 74 may optionally be held under tension, such that resection naturally occurs along the resection line 70 as the adjacent tissue is weakened by coagulation 72.

During tissue coagulation 72 and separation 80, there may be anatomical vessels 85, such as blood vessels, that traverse the resection line 70, as illustrated in **Fig. 8C**. Notably, because blood vessels are mostly composed of collagen, they will typically remain intact even through the surrounding tissue (e.g., the parenthymia of an organ) does separate, resulting in the skeletalization of the tissue. In this case, the ablation probe 12 may be used to seal the portion of the blood vessel that traverses the resection line 70. Using a separate device, such as scissors, the sealed portion of the blood vessel can then be transected 88, as illustrated in **Fig. 8D****.** The tissue coagulation 72 and separation 80 steps can be repeated until the unhealthy tissue portion 75 has been completely resected from the healthy tissue portion 73.

## Claims

1. A tissue ablation probe, comprising:
an elongated probe shaft having a proximal end and a distal end;
at least one fluid perfusion lumen longitudinally extending through the probe shaft;
a plurality of electrodes carried by the distal end of the probe shaft in a side-by-side arrangement, each of the electrodes including a porous structure in fluid communication with the at least one fluid perfusion lumen, each of the electrodes having a distal-facing surface **characterised in that** the distal-facing surfaces of the electrodes together form a continuous surface, the continuous surface being configured to conform to a surface of tissue to be subjected to the tissue ablation probe;
wherein the continuous surface is tapered with respect to a longitudinal axis of the probe shaft; and
wherein the porous structure of each of the electrodes is composed of an electrically conductive material.

2. The tissue ablation probe of claim 1, wherein the probe shaft comprises a plurality of tubes on which the plurality of electrodes are respectively mounted, and wherein the at least one fluid perfusion lumen comprises a plurality of fluid perfusion lumens longitudinally extending through the plurality of tubes.

3. The tissue ablation probe of any of claims 1 or 2, wherein the substantially continuous surface is a substantially planar surface.

4. The tissue ablation probe of any of claims 1-3, wherein the substantially continuous surface is a curved surface.

5. The tissue ablation probe of any of claims 1-4, wherein the plurality of electrodes are configured in a multi-polar arrangement.

6. The tissue ablation probe of any of claims 1-5, wherein the porous structure of each of the electrodes has pores with effective diameters in the range of 1-50 microns.

7. The tissue ablation probe of any of claims 1-6, wherein the porous structure of each of the electrodes has interconnecting pores.

8. The tissue ablation probe of any of claims 1-7, further comprising a connector assembly mounted to the proximal end of the probe shaft, wherein the connector assembly comprises at least one port in fluid communication with the at least one fluid perfusion lumen.

9. The tissue ablation probe of any of claims 1-8, wherein the plurality of electrodes include first and second electrodes configured in a bipolar arrangement.

## Patentansprüche

1. Gewebeablationssonde, umfassend:
einen ausgedehnten Sondenschaft mit einem proximalen Ende und einem distalen Ende;
wenigstens einen Fluidperfusionslumen, der sich längs durch den Sondenschaft erstreckt;
eine Vielzahl von Elektroden, die in einer Seite-an-Seite-Anordnung von dem distalen Ende des Sondenschaftes getragen werden, wobei jede der Elektroden eine poröse Struktur in fluider Kommunikation mit dem wenigstens einen Fluidperfusionslumen umfasst, wobei jede der Elektroden eine distal zugewandte Oberfläche aufweist,
**dadurch gekennzeichnet,**
**dass** die distal zugewandten Oberfächen der Elektroden zusammen eine kontinuierliche Oberfläche ausbilden, wobei die kontinuierliche Oberfläche dazu ausgebildet ist, zu einer Gewebeoberfläche zu passen, auf die mit der Gewebeablationssonde eingewirkt werden soll,
wobei die kontinuierliche Oberfläche sich hinsichtlich einer Längsachse des Sondenschaftes verjüngt, und
wobei die poröse Struktur von jeder der Elektroden ein elektrisch leitfähiges Material umfasst.

2. Gewebeablationssonde nach Anspruch 1, wobei der Sondenschaft eine Vielzahl von Röhrchen umfasst, auf denen die Vielzahl von Elektroden jeweils aufgebracht sind, und wobei der wenigstens eine Fluidperfusionslumen eine Vielzahl von Fluidperfusionslumen umfasst, die sich längs durch die Vielzahl von Röhrchen erstrecken.

3. Gewebeablationssonde nach Anspruch 1 oder Anspruch 2, wobei die im Wesentlichen kontinuierliche Oberfläche eine im Wesentlichen ebene Oberfläche ist.

4. Gewebeablationssonde nach einem der Anspräche 1 bis 3, wobei die im Wesentlichen kontinuierliche Oberfläche eine gebogene Oberfläche ist.

5. Gewebeablationssonde nach einem der Ansprüche 1 bis 4, wobei die Vielzahl von Elektroden in einer multipolaren Anordnung ausgebildet ist,

6. Gewebeablationssonde nach einem der Ansprüche 1 bis 5, wobei die poröse Struktur von jeder der Elektroden Poren aufweist, die effektive Durchmesser im Bereich von 1-50 Mikrometer aufweisen.

7. Gewebeablationssonde nach einem der Anspruchs 1 bis 6, wobei die poröse Struktur von jeder der Elektroden untereinander verbundene Poren aufweist.

8. Gewebeablationssonde nach einem der Ansprüche 1 bis 7, ferner umfassend eine Verbindungsanordnung, die an das proximale Ende des Sondenschaftes angebracht ist, wobei die Verbindungsanordnung wenigstens einen Anschluss in fluider Kommunikation mit dem wenigstens einen Fluidperfusionslumen umfasst.

9. Gewebeablationssonde nach einem der Ansprüche 1 bis 8, wobei die Vielzahl von Elektroden erste und zweite Elektroden umfasst, die in einer bipolaren Anordnung ausgebildet sind.

## Revendications

1. Sonde d'ablation de tissus, comprenant :
- une Lige de sonde allongée présentant une extrémité proximal et une extrémité distale ;
- au moins une lumière à perfusion de fluide s'étendant de manière longitudinale à travers la tige de sonde ;
- une pluralité d'électrodes portée par l'extrémité distale de la tige de sonde, disposées côte à côte, chacune des électrodes comportant une structure poreuse en communication de fluide avec ladite au moins une lumière à perfusion de fluide, chacune des électrodes présentant une surface à présentation distale, **caractérisée en ce que** les surfaces à présentation distale des électrodes forment ensemble une surface continue, celle-ci étant configurée pour se conformer à une surface de tissu devant être soumis à la sonde d'ablation de tissus ;
- dans laquelle la surface continue présente un profil effilé par rapport à un axe longitudinal de la tige de la sonde ; et
- dans laquelle la structure poreuse de chacune des électrodes est constituée d'un matériau conducteur d'électricité.

2. Sonde d'ablation de tissus selon la revendication 1, dans laquelle la tige de la sonde comprend une pluralité de tubes sur laquelle la pluralité d'électrodes est respectivement montée, et dans laquelle la au moins une lumière à perfusion de liquide comprend une pluralité de lumières à perfusion de liquide s'étendant de manière longitudinale à travers ladite pluralité de tubes.

3. Sonde d'ablation de tissus selon la revendication 1 ou la revendication 2, dans laquelle la surface sensiblement continue présente une surface sensiblement plane.

4. Sonde d'ablation de tissus selon l'une quelconque des revendications 1 à 3, dans laquelle la surface sensiblement continue présente une surface sensiblement courbe.

5. Sonde d'ablation de tissus selon l'une quelconque des revendications 1 à 4, dans laquelle la pluralité d'électrodes est configurée selon une disposition multipôle.

6. Sonde d'ablation de tissus selon l'une quelconque des revendications 1 à 5, dans laquelle la structure poreuse de chacune des électrodes présente des pores ayant des diamètres fonctionnels compris entre 1 et 50 microns.

7. Sonde d'ablation de tissus selon l'une quelconque des revendications 1 à 6, dans laquelle la structure poreuse de chacune des électrodes présente des pores interconnectées.

8. Sonde d'ablation de tissus selon l'une quelconque des revendications 1 à 7, comprenant en outre un ensemble à connecteurs monté à l'extrémité proximale de la tige de sonde, et dans laquelle l'ensemble à connecteurs comprend au moins un orifice en communication de fluide avec la au moins une lumière à perfusion de fluide.

9. Sonde d'ablation de tissus selon l'une quelconque des revendications 1 à 8, dans laquelle la pluralité d'électrodes comporte une première et une deuxième électrode configurées en disposition bipôle.
